# EUROPEAN PATENT SPECIFICATION

(11) **EP 4 198 144 B1**
(45) Date of publication and mention of the grant of the patent: **14.05.2025**
(21) Application number: 21855505.0
(22) Date of filing: 10.08.2021
(51) Int. Cl.: C08J 7/06, C08J 7/12, C12Q 1/6837

(54) **AMINO-MODIFIED CHIP, PREPARATION METHOD THEREFOR AND USE THEREOF**
AMINOMODIFIZIERTER CHIP, HERSTELLUNGSVERFAHREN DAFÜR UND VERWENDUNG DAVON
PUCE MODIFIÉE PAR DES AMINES, SON PROCÈDE DE PRÉPARATION ET SON UTILISATION

(30) Priority: 12.08.2020 CN 202010807921
(43) Date of publication of application: 21.06.2023
(73) Proprietor: GeneMind Biosciences Company Limited, Luohu District, Shenzhen, Guangdong 518000 (CN)
(72) Inventor: FENG, Diewen, Shenzhen, Guangdong 518000 (CN); LIN, Zhifeng, Shenzhen, Guangdong 518000 (CN); FAN, Jicai, Shenzhen, Guangdong 518000 (CN); LIU, Lei, Shenzhen, Guangdong 518000 (CN); CHEN, Fang, Shenzhen, Guangdong 518000 (CN); WANG, Qi, Shenzhen, Guangdong 518000 (CN)
(74) Representative: Schiweck Weinzierl Koch Patentanwälte Partnerschaft mbB
(86) International application number: PCT/CN2021/111672
(87) International publication number: WO 2022/033453

(56) References cited:
- WO-A1-2009/126259
- WO-A1-2016/123594
- WO-A2-2019/173695
- CN-A- 107 305 214
- CN-B- 101 643 321
- CN-B- 107 305 214
- "Recognition Receptors in Biosensors", 23 November 2009, SPRINGER NEW YORK, New York, NY, ISBN: 978-1-4419-0919-0, article VINCENT DUGAS ET AL: "Surface Sensitization Techniques and Recognition Receptors Immobilization on Biosensors and Microarrays", pages: 47 - 134, XP055521889, DOI: 10.1007/978-1-4419-0919-0_2
- SHIROSAKI ET AL.: "In vitro cytocompatibility of MG63 cells on chitosan-organosiloxane hybrid membranes", BIOMATERIALS, vol. 26, no. 5, 28 February 2005 (2005-02-28), XP025280443, DOI: 10.1016/j.biomaterials.2004.02.056
- LI, FENG ET AL.: "Construction of Bienzyme Biosensor based on Combination of One-Step Electrodeposition and Covalent-Coupled Sol-Gel Process", SCIENCE IN CHINA(SERIES B:CHEMISTRY), vol. 39, no. 7, 15 July 2009 (2009-07-15), pages 640 - 645, XP055900184

## Description

### TECHNICAL FIELD

The present disclosure relates to the technical field of chips, and particularly, to an amino-modified chip, preparation method thereof and use thereof.

### BACKGROUND

With the gradual implementation of Human Genome Project and the rapid development of related disciplines in molecular biology, more and more genome sequences of animals, plants and microorganisms have been determined, and gene sequence data has increased rapidly at an unprecedented rate. The gene chip (also called DNA chip, biochip) technology, created with the need of such scientific development, is used for preparing and/or analyzing biomolecules which specifically refer to probe molecules (not limited to nucleic acid sequences) that are immobilized on a support and then hybridized with labeled sample molecules. The quantity and sequence information of the sample molecules are obtained by detecting the hybridization signal intensity of each probe molecule.

Chips are generally made by polymer-coated substrates. The analysis and/or preparation of molecules, such as some methods for nucleic acid sequencing, depend on the bonding of nucleic acid strands to the surface of the chip substrate, and the sequence of the bonded nucleic acid strand is then determined by a number of different methods well known in the art. Existing substrates are generally flow cells. Process for coating a flow cell may include transferring the polymerized mixture into channels on the flow cell and incubating for a fixed time. The process is simple and can produce reliable coatings that are always able to support all downstream chemical processing procedures including bridge amplification and sequencing.

CN101643321B discloses a high-polymer three-dimensional amino-group substrate as well as preparation method and application thereof.

WO2019/173695 A2 discloses to a microarray based multiplex pathogen analysis an uses thereof.

WO2009/126259 A1 discloses to a surface for label independent detection and method thereof.

Dugas et al., Recognition Receptors in Biosensors" November 2009, pages 47-134, discloses surface sensitization techniques and recognition receptors immobilization on biosensors and microarrays.

CN107305214B discloses a manufacturing method for a hard micro-fluid chip.

However, existing chips are in need of further improvement, since they still have many defects or limitations limiting the applications thereof.

### SUMMARY

Based on this, it is necessary to provide an amino-modified chip. The amino-modified chip can carry a probe load of a higher density, and better meet the requirements of continuously developing biomolecule preparation and/or analysis with good stability

Examples of the present disclosure provide an amino-modified chip, containing:
a substrate modified with an epoxy group; and
a polymer attached to the substrate via the epoxy group, where at least one structural unit of the polymer contains an amino group, and the amino group is a primary amino group and/or a secondary amino group.

In some embodiments, in the polymer, each of the structural unit contains an average of 0.05 to 10 identical or different amino groups.

In some embodiments, in the polymer, each of the structural unit contains an average of 1 to 5 identical or different amino groups.

In some embodiments, the polymer has a degree of polymerization in a range of 10 to 5000.

In some embodiments, in the polymer the structural unit contains at least one of

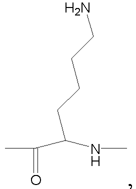

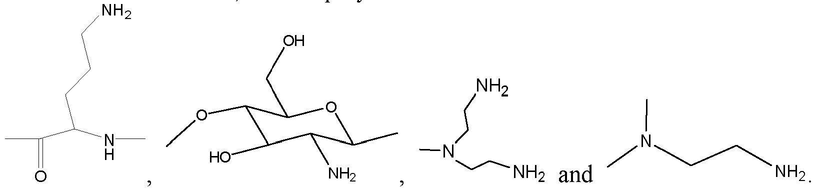

In some embodiments, the polymer is selected from at least one of polylysine, polyornithine, chitosan, a polyamidoamine dendrimer and polyethyleneimine.

, The chip further contains a probe attached to the polymer.

The probe is bonded to the polymer via a linker compound, and the linker compound has a molecular structure containing a first linker compound and a second linker compound; the first linker compound is bonded to the probe, and the second linker compound is attached to the polymer through the amino group.

The second linker compound is selected from at least one of an -NHS group, an epoxy group and an isocyanate group.

The second linker compound is selected from at least one of an -NHS group, an epoxy group and an isocyanate group, wherein the second linker compound is made of NHS-PEGₙ-DBCO or NHS-PEGₙ-N₃, wherein n is 3 to 2000, and group -NHS in NHS-PEGₙ-DBCO or NHS-PEGₙ-N₃ is attached to the amino group via reaction.

In some embodiments, the linker compound is NHS-PEGₙ-DBCO or NHS-PEGₙ-N₃, where n is 3 to 2000, group -NHS in NHS-PEGₙ-DBCO or NHS-PEGₙ-N₃ is attached to the amino group via reaction.

In some embodiments, the probe is modified with group -DBCO or group -N₃; the bonding is a covalent bonding between group -DBCO and group -N₃. . A method for preparing a chip, comprising:
(1) acquiring a substrate modified with an epoxy group;
(2) attaching a polymer to the substrate via reaction, wherein at least one structural unit of the polymer comprises an amino group, and the amino group is a primary amino group or a secondary amino group; and
(3) attaching a probe to the polymer, wherein the probe in step (3) is attached to the polymer via a linker compound, and the linker compound has a molecular structure comprising a first linker compound and a second linker compound; the first linker compound is bonded to the probe, and the second linker compound is attached to the polymer through the amino group,
wherein the second linker compound is selected from at least one of an -NHS group, an epoxy group and an isocyanate group, the linker compound is made of NHS-PEGₙ-DBCO or NHS-PEGₙ-N₃, wherein n is 3 to 2000, and group -NHS in NHS-PEGₙ-DBCO or NHS-PEGₙ-N₃ is attached to the amino group via reaction.

Examples of the present disclosure further provide a method for preparing a chip, containing:
(1) acquiring a substrate modified with an epoxy group; and
(2) attaching a polymer to the substrate via reaction, where at least one structural unit of the polymer contains an amino group, and the amino group is a primary amino group or a secondary amino group.

In some embodiments, in the polymer, each of the structural unit contains an average of 0.05 to 10 identical or different amino groups
and
(3) attaching a probe to the polymer, wherein the probe in step (3) is attached to the polymer via a linker compound, and the linker compound has a molecular structure comprising a first linker compound and a second linker compound; the first linker compound is bonded to the probe, and the second linker compound is attached to the polymer through the amino group,
wherein the second linker compound is selected from at least one of an -NHS group, an epoxy group and an isocyanate group, the linker compound is made of NHS-PEGₙ-DBCO or NHS-PEGₙ-N₃, wherein n is 3 to 2000, and group -NHS in NHS-PEGₙ-DBCO or NHS-PEGₙ-N₃ is attached to the amino group via reaction.

In some embodiments, in the polymer, each of the structural unit contains an average of 1 to 5 identical or different amino groups.

In some embodiments, the polymer has a degree of polymerization in a range of 10 to 5000.

In some embodiments, in the polymer the structural unit contains at least one of

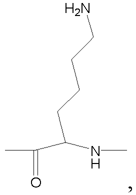

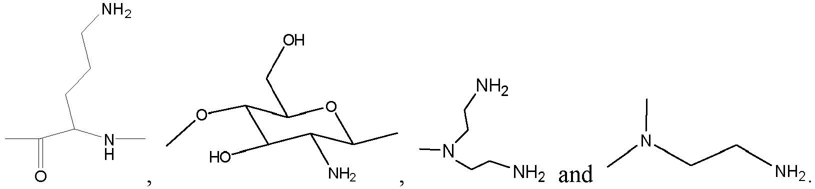

In some embodiments, the polymer is selected from at least one of polylysine, polyornithine, chitosan, a polyamidoamine dendrimer and polyethyleneimine.

In some embodiments, in step (2), the attaching via reaction is conducted by contacting the polymer with the substrate in an alkaline solution at pH 8.5 to 10.

In some embodiments, in step (2), the grating via reaction is conducted at a reaction temperature of 37 to 55 °C for 3 to 24 h.

In some embodiments, the method further contains step (3): attaching a probe to the polymer.

In some embodiments, the probe is attached to the polymer via a linker compound, and the linker compound has a molecular structure containing a first linker compound and a second linker compound; the first linker compound is bonded to the probe, and the second linker compound is attached to the polymer through the amino group.

In some embodiments, the second linker compound is selected from at least one of an -NHS group, an epoxy group and an isocyanate group.

In some embodiments, the linker compound is NHS-PEGₙ-DBCO or NHS-PEGₙ-N₃, where n is 3 to 2000, group -NHS in NHS-PEGₙ-DBCO or NHS-PEGₙ-N₃ is attached to the amino group via reaction.

In some embodiments, the probe is modified with group -DBCO or group -N₃; the bonding is a covalent bonding between group -DBCO and group -N₃.

In some embodiments, the attaching via reaction is conducted by contacting the linker compound with the polymer in an alkaline solution at pH 7 to 9.

In some embodiments, the attaching via reaction is conducted at room temperature for 30 to 90 min. In some embodiments, the covalent bonding is conducted by contacting the probe with the linker compound in an alkaline solution at pH 7 to 8.

In some embodiments, the alkaline solution for covalent bonding contains a surfactant selected from at least one of tetradecyltrimethylammonium bromide, cetyltrimethylammonium bromide and dodecyltrimethylammonium bromide.

Embodiments of the present disclosure further provides use of the chip as described above, or a chip prepared by the method as described above in preparation or analysis of a biomolecule.

The amino-modified chip disclosed in one embodiment of the present disclosure has a substrate modified with the specific active group, and the substrate is attached to a polymer containing a primary amino group and/or a secondary amino group via reaction. The polymer can form a modified surface with a higher density and a high reactivity for attaching a probe. The amino-modified chip can carry a probe load of a higher density, and better meet the requirements of continuously developing biomolecule preparation and/or analysis with good stability. The method for preparing a chip disclosed in another embodiment of the present disclosure can achieve the attaching of the probe without controlling reaction conditions strictly, making the preparing process for the chip simple, easy to control and favorable for popularization and application.

The invention is further directed to the use of the chip or a chip prepared by the method as discussed above in preparation or analysis of a biomolecule.

### BRIEF DESCRIPTION OF THE DRAWINGS

FIG 1 is a schematic illustrating a preparation process of a chip in the examples, where X represents the polymer, and primer represents the primer/probe;
FIG 2 is a graph illustrating probe density measurement results of chips prepared in Examples 1-5;
FIG 3 is a graph illustrating DNA cluster detection results generated by the chip prepared in Example 1;
FIG 4 is a graph illustrating DNA cluster detection results generated by the chip prepared in Example 2;
FIG 5 is a graph illustrating DNA cluster detection results generated by the chip prepared in Example 3;
FIG 6 is a graph illustrating DNA cluster detection results generated by the chip prepared in Example 4;
FIG 7 is a graph illustrating DNA cluster detection results generated by the chip prepared in Example 5; and
FIG 8 is a structural schematic of the DNA library used in Example 7.

### DETAILED DESCRIPTION

The amino-modified chip of the present disclosure, the method for preparing the same and the use thereof are described in further details below with reference to specific examples. The present disclosure can be implemented in many different forms and is not limited to the embodiments described herein. In contrast, these embodiments are provided for a thorough and complete understanding of the present disclosure.

Unless defined otherwise, all technical and scientific terms used herein have the same meaning as commonly understood by those skilled in the art to which the present disclosure belongs. The terms used in the specification of the present disclosure herein are for the purpose of describing specific examples only and are not intended to limit the scope of the present disclosure. As used herein, the term "and/or" includes any and all combinations of one or more of the associated listed items.

The chip described herein may be a substrate to which the polymer is attached, or a substrate where a probe is further attached to the polymer. Materials for the substrate are not specified, and the substrate is formed of at least one of glass, silicon wafer, plastic, gel and nylon film.

The term "attached to" or "modified with" as used herein may refer to a direct attach to or modification with an object, or may refer to a further attach to or modification with the object via another transition group.

The amino group as used herein refers to a structural feature having a formula -N(X)₂, where each "X" is independently H, a substituted or unsubstituted alkyl, a substituted or unsubstituted cycloalkyl, a substituted or unsubstituted heterocyclyl, and the like. It will be appreciated that in the context of the present disclosure, at least one X is H. Non-limiting types of the amino group include -NH₂, -NH(alkyl), -NH(cycloalkyl), -NH(heterocyclyl) and -NH(aryl).

The term "alkyl" refers to a saturated hydrocarbon containing a primary (normal) carbon atom, a secondary carbon atom, a tertiary carbon atom, a quaternary carbon atom, or a combination thereof. Suitable examples include, but are not limited to: methyl (Me, -CH₃), ethyl (Et, -CH₂CH₃), 1-propyl (*n*-Pr, *n*-propyl, -CH₂CH₂CH₃), 2-propyl (*i*-Pr, *i*-propyl, -CH(CH₃)₂), 1-butyl (*n*-Bu, *n*-butyl, -CH₂CH₂CH₂CH₃), 2-methyl-1-propyl (*i*-Bu, i-butyl, -CH₂CH(CH₃)₂), 2-butyl (*s*-Bu, *s*-butyl, -CH(CH₃)CH₂CH₃), 2-methyl-2-propyl (*t*-Bu, *t*-butyl, -C(CH₃)₃), 1-pentyl (n-pentyl, -CH₂CH₂CH₂CH₂CH₃), 2-pentyl (-CH(CH₃)CH₂CH₂CH₃), 3-pentyl (-CH(CH₂CH₃)₂), 2-methyl-2-butyl (-C(CH₃)₂CH₂CH₃), 3-methyl-2-butyl (-CH(CH₃)CH(CH₃)₂), 3-methyl-1-butyl (-CH₂CH₂CH(CH₃)₂), 2-methyl-1-butyl(-CH₂CH(CH₃)CH₂CH₃), 1-hexyl (-CH₂CH₂CH₂CH₂CH₂CH₃), 2-hexyl (-CH(CH₃)CH₂CH₂CH₂CH₃), 3-hexyl (-CH(CH₂CH₃)(CH₂CH₂CH₃)), 2-methyl-2-pentyl (-C(CH₃)₂CH₂CH₂CH₃), 3-methyl-2-pentyl (-CH(CH₃)CH(CH₃)CH₂CH₃), 4-methyl-2-pentyl (-CH(CH₃)CH₂CH(CH₃)₂), 3-methyl-3-pentyl (-C(CH₃)(CH₂CH₃)₂), 2-methyl-3-pentyl (-CH(CH₂CH₃)CH(CH₃)₂), 2,3-dimethyl-2-butyl (-C(CH₃)₂CH(CH₃)₂), 3,3-dimethyl-2-butyl (-CH(CH₃)C(CH₃)₃ and octyl (-(CH₂)₇CH₃).

The term "cycloalkyl" refers to a non-aromatic hydrocarbon containing ring carbon atoms and may be a monocyclic, spirocycloalkyl or bridged cycloalkyl group. Suitable examples include, but are not limited to: cyclopropyl, cyclobutyl, cyclopentyl, cyclohexyl and cycloheptyl. In addition, "cycloalkyl" may also contain one or more double bonds, and representative examples of cycloalkyl groups containing double bonds include cyclopentenyl, cyclohexenyl, cyclohexadienyl and cyclobutadienyl.

The term "aryl" refers to an aromatic hydrocarbon group derived by removing one hydrogen atom from an aromatic cyclic compound, and may be a monocyclic aryl, fused cyclic aryl or polycyclic aryl. For polycyclic species, at least one of the rings is an aromatic ring system. Suitable examples include, but are not limited to: benzene, biphenyl, naphthalene, anthracene, phenanthrene, perylene, triphenylene, and derivatives thereof.

The term "heterocyclyl" refers to a cycloalkyl with at least one carbon atom replaced by a non-carbon atom, which may be an N atom, an O atom, an S atom, etc. The heterocyclyl may be a saturated ring or a partially unsaturated ring. Suitable examples include, but are not limited to: dihydropyridinyl, tetrahydropyridyl (piperidinyl), tetrahydrothienyl, thiooxidized tetrahydrothienyl, tetrahydrofuranyl, tetrahydroquinolinyl, tetrahydroisoquinolinyl and indolinyl.

The term "structural unit" refers to a unit structure formed by one of the monomers in the polymer. The polymer may be polymerized from one monomer or two or more different monomers, and the "structural units" may be identical or different.

The relationship between degree of polymerization and number of generation for polyamidoamine dendrimer: the degree of polymerization = 2 ^ (number of generation + 1) - 1. For example, for PamamDendrimer generation 3.0, the degree of polymerization is 15.

Examples of the present disclosure provide an amino-modified chip, containing:
a substrate modified with an epoxy group; and
a polymer attached to the substrate via the epoxy group, where at least one structural unit of the polymer contains an amino group, and the amino group is a primary amino group and/or a secondary amino group.

It will be appreciated that the chip can be used for genetic testing, or can be used for protein, polypeptide or other detections.

In one of the specific examples, in the polymer, each of the structural unit contains an average of 0.05 to 10 identical or different amino groups. It will be appreciated that the structural unit may or may not contain amino groups. The term "average" refers to a mean value obtained by dividing the total number of amino groups by the total number of the structural units. When the average number of amino groups in each structural unit is less than 1, the polymer is a copolymer obtained by polymerizing two or more monomers, at least one of which does not contain an amino group. For example, in the polymer each of the structural unit may contain an average of 0.5 amino groups, and the polymer may be composed of 19 structural units free of amino group and 1 structural unit containing amino groups (containing 10 amino groups). The number of amino groups can be controlled by controlling the combination of monomers in the polymerization.

Preferably, in the polymer, each of the structural unit contains an average of 1 to 5 amino groups. It is found that the number of amino groups in the polymer is associated with the reactivity of the amino groups, which directly influences the density/number of linkages, such as probes, between the amino groups and linkers connected to the polymer. In one example, the probes are linked to the surface of the chip through the linker compounds and the amino groups of the polymer. Controlling the average number of amino groups contained in each structural unit of the polymer at 1 to 5 can well regulate the linkage density/number of the probes, thus optimizing the regulation and control of the probe density. Meanwhile, the probes can be more easily attached to the polymer through the linker compounds with a higher probe density.

In one of the specific examples, the polymer has a degree of polymerization in a range of 10 to 5000. In another one of the specific examples, the polymer has a molecular weight of 30 kD to 300 kD. A higher molecular weight may result in a higher density of nucleic acid strand that can be attached. However, at the same time, it may increase the effectiveness of immobilization of the polymer on the surface of the substrate. Also, molecules with an excessively high molecular weight have undesirable effects in terms of solubility, reaction efficiency, and the like. Therefore, in the examples of the present disclosure, the molecular weight of the polymer is preferably 30 kD to 300 kD. For the polyamidoamine dendrimer, e.g., the PamamDendrimer, the number of generations may be 3 to 11.

Preferably, the polymer contains identical or different structural units, and may be commercially available or customized, as long as the requirements of the present application for the number of amino groups contained in the polymer and/or the degree of polymerization of the polymer are met. In one of the specific examples, in the polymer the structural unit contains at least one of

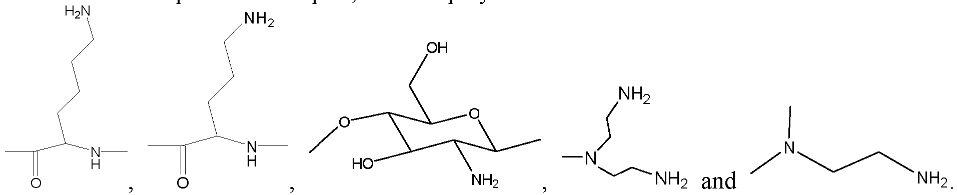

Specifically, in one example, the polymer contains identical structural units. For example, the polymer is a copolymer containing identical structural units of 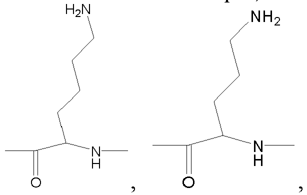 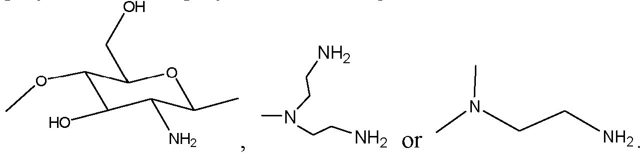 In another example, the polymer contains different structural units. For example, the polymer is a copolymer containing different structural units of 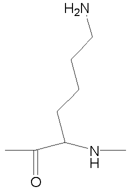 and 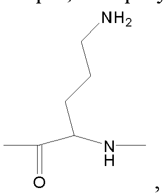 as disclosed in Kousaku Ohkawaa, "Biodegradation of Ornithine-Containing Polylysine Hydrogels", Biomaterials 19(1998): 1855-1860, including copolymers of ornithine and lysine polymerized at different ratios, or the polymer is a copolymer containing different structural units of 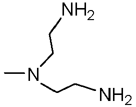 and

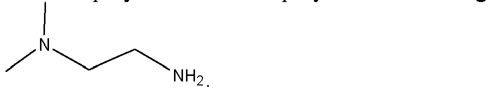

Preferably, the polymer is selected from at least one of polylysine, polyornithine, chitosan, a polyamidoamine dendrimer and polyethyleneimine.

In one of the specific examples, the chip further contains a probe attached onto the polymer. The probe binds to a target molecule and thus captures the target molecule. For different purposes, different probes may be selected. For example, oligonucleotide fragments, polypeptide sequences, probes containing oligonucleotide fragments, or probes containing polypeptide sequences can be selected. Meanwhile, the probes can selectively label fluorescent detection molecules, antigens, biotin, streptavidin, or other detection molecules.

In one of the specific examples, the probe may be a nucleic acid strand and/or a polypeptide.

In one of the specific examples, the probe is attached onto the polymer via a linker compound, and the linker compound has a molecular structure containing a first linker compound and a second linker compound; the first linker compound at one end is bonded to the nucleic acid strand probe, and the second linker compound at the other end is attached to the polymer through the amino group. Moreover, the second linker compound is selected from at least one of an -NHS group, an epoxy group and an isocyanate group.

In one of the specific examples, the linker compound is made of NHS-PEGₙ-DBCO or NHS-PEGₙ-N₃, where n is 3 to 2000, group -NHS in NHS-PEGₙ-DBCO or NHS-PEGₙ-N₃ is attached to the amino group via reaction.

Preferably, the probe is modified with group -DBCO or group -N₃; the bonding is a covalent bonding between group -DBCO and group -N₃. NHS is an abbreviation for succinimidyl ester, PEG for polyethylene glycol, DBCO for diphenylcyclooctyne, N₃ for azide. The linkage between the nucleic acid strand and the polymer via the linker compound can, on one hand, allow NHS to be stably attached to the amino, and on the other hand, allow DBCO or N₃ to be subjected to a Click reaction with a nucleic acid strand modified with N₃ or DBCO under mild conditions of room temperature and no catalyst with high efficiency.

Examples of the present disclosure further provide a method for preparing a chip, containing:
(1) acquiring a substrate modified with an epoxy group; and
(2) attaching a polymer to the substrate via reaction, where at least one structural unit of the polymer contains an amino group, and the amino group is a primary amino group or a secondary amino group.

In the above method, further definitions of the polymer are the same as those for the chip described above and are not repeated hereinafter.

Specifically, step (1) is a substrate acquisition procedure. The acquisition can be a direct purchase, that is, acquiring a substrate modified with an epoxy group by direct purchase; or the acquisition can be self-making, where the process for self-making can be modifying a compound containing an epoxy group on the carrier by a solution reaction or plasma spray-coating.

The compound containing an epoxy group may be selected from an epoxy silane, such as 3-(2,3-glycidoxy)propyltrimethoxysilane. Additionally, in one of the specific examples, the material of the carrier is at least one of glass, silicon wafer, plastic, gel and nylon film.

In one of the specific examples, the reaction conditions for modifying the compound containing an epoxy group on the carrier by a solution reaction include: a reaction temperature at room temperature, and a reaction time of 1 to 8 h. Moreover, after the reaction, the mixture is dried at 80 to 150 °C.

It will be appreciated that the carrier may require extensive washing and activation prior to the solution reaction modification. For example, the carrier surface is alternately washed with an alcohol solvent and water under ultrasonic conditions and activated with an alkaline solution. The alkaline solution activation can be conducted by processing, e.g., in a 0.1 M to 2 M aqueous NaOH solution for 1 to 20 min.

Specifically, step (2) is a polymer attaching procedure. The epoxy group on the substrate is modified, and in step (2), the amino group and the epoxy group are subjected to a attaching via reaction.

Preferably, the attaching via reaction is conducted by contacting the polymer with the substrate in an alkaline solution at pH 8.5 to 10. The pH values include, but are not limited to: 8.5, 8.8, 9, 9.16, 9.2, 9.5 and 10.

Preferably, the alkaline solution at pH 8.5 to 10 is selected from at least one of a phosphate buffer, a carbonate buffer and a borate buffer. Furthermore, the concentration of the solute pair in the buffer at pH 8.5 to 10 is 10 to 300 mM. The concentrations of the solute pairs include, but are not limited to: 10 mM, 50 mM, 100 mM, 120 mM, 150 mM, 190 mM, 195 mM, 200 mM, 205 mM, 210 mM, 220 mM, 250 mM and 300 mM.

Preferably, a carbonate buffer at pH 9.16 is used as the reaction solvent, where the concentration of the solute pair is 200 mM.

Additionally, in step (2), the attaching via reaction is conducted at a reaction temperature of 37 to 55 °C for 3 to 24 h. The combination of reaction temperature and reaction time (temperature × time) includes, but is not limited to: 37 °C × 14 h, 37 °C × 16 h, 37 °C × 18 h, 37 °C × 20 h, 37 °C × 24 h, 40 °C × 12 h, 42 °C × 18 h, 45 °C × 10 h, 50 °C × 8 h, 55 °C × 5 hand 55 °C × 3 h.

Preferably, the reaction temperature is 37 °C and the reaction time is 16 h.

Preferably, the method for preparing the chip described above further contains step (3): attaching a probe to the polymer. Step (3) is a probe attaching procedure.

In one of the specific examples, in step (3), the probe is attached to the polymer via a linker compound, and the linker compound has a molecular structure containing a first linker compound and a second linker compound; the first linker compound is bonded to the probe, and the second linker compound is attached to the polymer through the amino group. More specifically, the second linker compound is selected from at least one of an -NHS group, an epoxy group and an isocyanate group.

Preferably, step (3) can be conducted in two procedures:
(3-1) subjecting a compound providing the linker compound and an amino group of the polymer to a attaching reaction; and
(3-2) bonding a product of the attaching reaction to a probe modified by an active group.

In one of the specific examples, the linker compound is made of NHS-PEGₙ-DBCO or NHS-PEGₙ-N₃, where n is 3 to 2000, group -NHS in NHS-PEGₙ-DBCO or NHS-PEGₙ-N₃ is attached to the amino group via reaction. The linkage between the probe and the polymer via the linker compound can, on one hand, allow NHS to be stably attached to the amino, and on the other hand, allow DBCO or N₃ to be subjected to a click reaction with a probe modified with N₃ or DBCO under mild conditions of room temperature and no catalyst with high efficiency.

Preferably, the active group-modified probe is modified with group -DBCO or group -N₃; the bonding is a covalent bonding between group -DBCO and group -N₃. More specifically, the covalent bonding is a click reaction.

In one of the specific examples, the active group-modified probe is a nucleic acid strand modified by the active group at the 5' end. It will be appreciated that if the linker compound is prepared from NHS-PEGₙ-DBCO, i.e., the group for bonding is -DBCO, the reactive group for 5' modification is -N₃ functional group; if the linker compound is prepared from NHS-PEGₙ-N₃, i.e., the group for bonding is -N₃, the reactive group for 5' modification is -DBCO functional group.

In one of the specific examples, in step (3-1), the concentration of the compound for providing the linker compound in the reaction system is 50 µM to 5 mM. The concentrations include, but are not limited to: 50 µM, 100 µM, 500 µM, 800 µM, 1 mM, 1.2 mM, 1.5 mM, 2 mM and 5 mM.

In one of the specific examples, in step (3-1), the attaching via reaction is conducted by contacting the linker compound with the polymer in an alkaline solution at pH 7 to 9. Specifically, the pH values include, but are not limited to: 7, 7.3, 7.5, 7.7, 7.8, 7.9, 8, 8.1, 8.2, 8.3, 8.4 and 8.5. More specifically, the alkaline solution is selected from at least one of a phosphate buffer and a carbonate buffer.

In one of the specific examples, the concentration of the solute pair in the phosphate buffer or the carbonate buffer is 10 mM to 300 mM. The concentrations of the solute pairs include, but are not limited to: 10 mM, 50 mM, 80 mM, 90 mM, 95 mM, 98 mM, 100 mM, 102 mM, 105 mM, 110 mM, 150 mM, 200 mM, 210 mM, 220 mM, 250 mM and 300 mM.

Preferably, a phosphate buffer at pH 8 is used as the reaction solvent for the attaching via reaction, where the concentration of the solute pair is 100 mM. In another one of the specific examples, a carbonate buffer at pH 8.3 is used as the reaction solvent for the attaching via reaction, where the concentration of the solute pair is 100 mM.

In one of the specific examples, in step (3-1), the attaching via reaction is conducted at room temperature for 30 to 90 min. The reaction times include, but are not limited to: 30 min, 40 min, 50 min, 55 min, 58 min, 60 min, 62 min, 65 min, 70 min, 80 min and 90 min.

In one of the specific examples, in step (3-2), the covalent bonding is conducted by contacting the probe with the linker compound in an alkaline solution at pH 7 to 8. Specifically, the pH values include, but are not limited to: 7, 7.3, 7.5, 7.7, 7.8, 7.9 and 8. More specifically, the alkaline solution is a citrate buffer. More specifically, the covalent bonding is conducted at room temperature.

In one of the specific examples, the alkaline solution contains a surfactant selected from at least one of tetradecyltrimethylammonium bromide (TTAB), cetyltrimethylammonium bromide (CTAB) and dodecyltrimethylammonium bromide (DTAB). The surfactant is used to facilitate the sedimentation of the nucleic acid strands on the surface.

Preferably, the covalent bonding is conducted by contacting the probe with the linker compound in a citrate buffer containing 1 mM TTAB at room temperature.

Additionally, in various applications, excessive functional groups, such as -NH₂, -DBCO, -N₃, etc., may be optionally blocked before or after bonding to the probe.

Specifically, in one of the specific examples, the method further containing: conducting a blocking process using at least one of NHS-(PEG)ₙ, acetic anhydride, DBCO-benzoic acid and azidobenzoic acid, where n = 3 to 2000. More specifically, the blocking of -NH₂ can be conducted using NHS-(PEG)ₙ (n can be 3 to 2000), acetic anhydride, etc.; specifically, for example, -NH₂ may be blocked using 1 µL of acetic anhydride + 1.75 µL of N,N-diisopropylethylamine (DIPEA) in formamide. The blocking of -N₃ can be conducted using DBCO-benzoic acid. The blocking of -DBCO can be conducted using azidobenzoic acid.

Examples of the present disclosure further provides use of the chip as described above, or a chip prepared by the method as described above in preparation or analysis of a biomolecule. Specifically, the preparation or analysis of the biomolecule can be, for example, nucleic acid sequencing, library hybridization, generation of DNA clusters, and the like, and can also be, for example, protein detection, polypeptide detection, and the like.

In the following specific examples provided below, unless otherwise indicated, all experimental materials are either available from commercial suppliers (e.g., Aladdin^{™}, Sigma^{™}, Sangon^{™}, etc.) or prepared in house or by a contractor in accordance with the structural formula and molecular weight information provided in the examples.

Polyamidoamine dendrimers (PamamDendrimer), or Pamam Dendrimer generation 3.0 (abbreviated as PD3.0), having a molecular weight of 6905.84 and the following molecular formula: 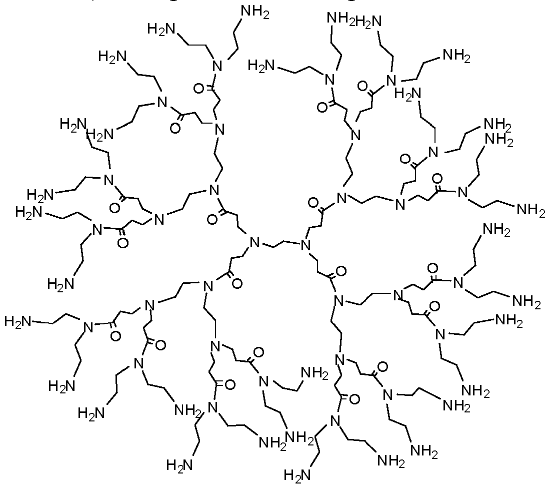 NHS-PEG₄-N₃ and NHS-PEG₄-DBCO, having the following molecular formulas: 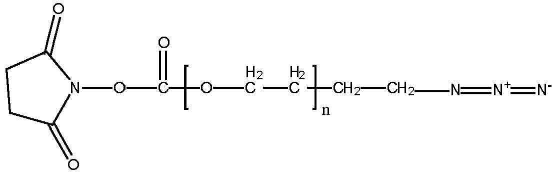 (NHS-PEG₄-N₃) 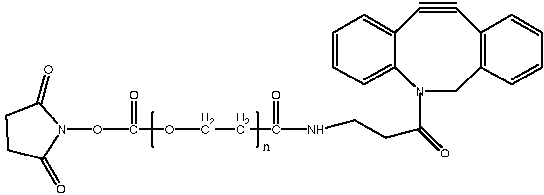 (NHS-PEG₄-DBCO) Anhydrous dimethyl sulfoxide (DMSO), purchased from Sigma^{™};
Polyethyleneimine (PEI), having a molecular weight of 60 kD and the following molecular formula: 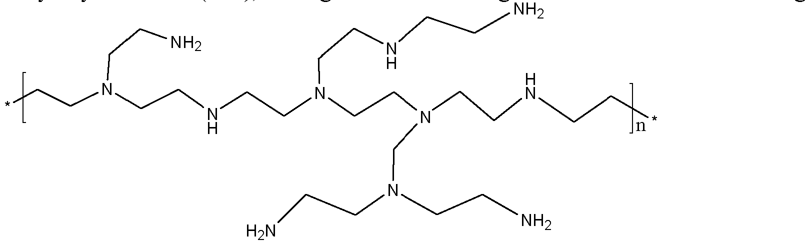
Polylysine (PLL), having a molecular weight of 150 to 300 kD and the following molecular formula: 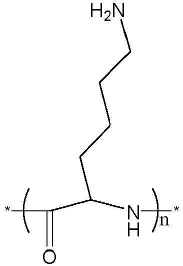
Polyornithine (PLO), having a molecular weight of 30 to 70 kD and the following molecular formula: 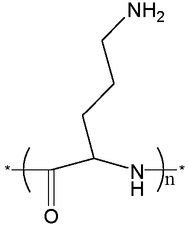
Chitosan, having a molecular weight of 100 kD and the following molecular formula: 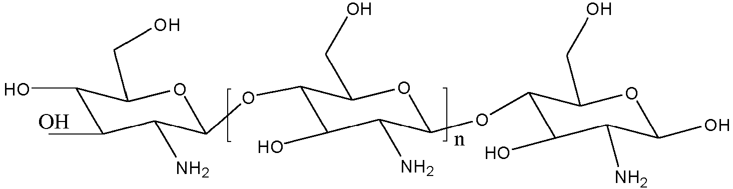
Sodium bicarbonate and sodium carbonate, purchased from Sigma^{™};
Tetradecyltrimethylammonium bromide (TTAB), purchased from Sigma^{™};
Disodium hydrogen phosphate and sodium dihydrogen phosphate, purchased from Sigma^{™};
20× SSC, purchased from Sangon^{™}; Hepes, purchased from Sigma^{™}; Tween 20, purchased from Sigma^{™};
Sodium dodecyl sulfate (SDS), purchased from Aladdin^{™};
SC: 0.2 M sodium carbonate, pH = 9.16 (25 °C);
PC: 0.1 M sodium phosphate, pH = 8.0 (25 °C);
Immobilization solution: 3× SSC + 1 mM TTAB (tetradecyltrimethylammonium bromide);
Washing solution 1: 150 mM Hepes + 0.1% Tween 20 + 0.1% SDS;
RI02: 3× SSC, pH = 7.2 to 7.4.

### Example 1

The specific procedures for coating the epoxy-modified substrate with PLO (polyornithine) and modifying the PLO-coated substrate with functional groups are as follows:
1. The 0.1% PLO solution was stored at room temperature for 15 min. 200 µL of SC buffer solution was added into 200 µL of the PLO solution. The mixture was well mixed by vortex, and centrifuged for 2 s.
2. The solution prepared in step 1 was introduced into lanes of an epoxy-modified substrate with 25 µL of solution into each lane. The lane ports were sealed (to prevent the solution from volatilizing). The substrate was incubated at 37 °C for 16 h.
3. After the reaction, when the substrate returned to room temperature, the substrate was washed with washing solution 1 and RI02, each 1 mL, on a fluid instrument. The parameters of the fluid instrument were: reagent amount = 1000, speed = 10, circle = 1, delay = 0, number = 1.
4. The PLO-coated substrate was soaked in RI02 solution for storage at 4 °C for 1 week.
5. The PLO-coated substrate was modified with functional groups, which refer to groups that can participate in subsequent reactions, and may be -N₃, -DBCO and the like. In this example, the functional group was -N₃. The specific procedures of the functional group modification are as follows:
   1) An NHS-PEG₄-N₃ solution with a final concentration of 100 mM was prepared with the solvent being anhydrous DMSO (anhydrous dimethyl sulfoxide);
   2) A solution containing NHS-PEG₄-N₃ was introduced into the lanes of the PLO-coated substrate:
      On the fluid instrument, liquid in the lanes of the substrate was drained, the lanes were rinsed with 1 mL of PC buffer per lane, and the liquid in the lanes was drained after rinsing;
   3) 396 µL of PC buffer and 4 µL of a 100 mM NHS-PEG₄-N₃ solution were mixed. The mixture was vortexed for mixing, and then centrifuged for 2 s;
   4) The solution prepared in the step 3) was immediately introduced into lanes of the substrate with 25 µL per lane. The substrate was incubated for 1 h at room temperature;
   5) After the reaction, the substrate was washed with washing solution 1, RI02 and ultrapure water, each 1 mL, on the fluid instrument;
   6) The liquid in the lanes of the substrate was drained on the fluid instrument. 497.25 µL of formamide was added into a 1.5-mL centrifuge tube before 1.75 µL of DIPEA and 1 µL of acetic anhydride were sequentially added. The mixture was vortexed for mixing, centrifuged for 2 s, and immediately introduced into the lanes of the substrate with 25 µL per lane. The substrate was incubated for 15 min at room temperature. After the reaction, the substrate was washed with washing solution 1, RI02 and ultrapure water, each 1 mL, on the fluid instrument.
6. Probe immobilization:
   1) On the fluid instrument, liquid in the lanes of the substrate was drained, the lanes were rinsed with 1 mL of immobilization solution per lane, and the liquid in the lanes was drained after rinsing;
   2) 360 µL of the immobilization solution and 40 µL of a 100 µM A30 (SEQ ID No. 1) solution were mixed. The mixture was vortexed for mixing, centrifuged for 2 s, and immediately introduced into the lanes of the substrate with 25 µL per lane. The substrate was incubated for 16 h at 37 °C;
   3) After the reaction, when the substrate returned to room temperature, the substrate was washed with washing solution 1 and RI02, each 1 mL, on a fluid instrument.

The functional group-modified substrate can be soaked in RI02 solution for storage at 4 °C for 1 year.

### Example 2

The specific procedures for coating the epoxy-modified substrate with PLL (polylysine) and modifying the PLL-coated substrate with functional groups are as follows:
1. The 0.1% PLL solution was stored at room temperature for 15 min. 200 µL of SC buffer solution was added into 200 µL of the PLL solution. The mixture was well mixed by vortex, and centrifuged for 2 s.
2. The solution prepared in step 1 was introduced into lanes of an epoxy-modified substrate with 25 µL of solution into each lane. The lane ports were sealed (to prevent the solution from volatilizing). The substrate was incubated at 37 °C for 16 h.
3. After the reaction, when the substrate returned to room temperature, the substrate was washed with washing solution 1 and RI02, each 1 mL, on a fluid instrument. The parameters of the fluid instrument were: reagent amount = 1000, speed = 10, circle = 1, delay = 0, number = 1.
4. The PLL-coated substrate was soaked in RI02 solution for storage at 4 °C for 1 week.
5. The PLL-coated substrate was modified with functional groups, which refer to groups that can participate in subsequent reactions, and may be -N₃, -DBCO and the like. In this example, the functional group was -N₃. The specific procedures of the functional group modification are as follows:
   1) An NHS-PEG₄-N₃ solution with a final concentration of 100 mM was prepared with the solvent being anhydrous DMSO (anhydrous dimethyl sulfoxide);
   2) A solution containing NHS-PEG₄-N₃ was introduced into the lanes of the PLL-coated substrate. On the fluid instrument, liquid in the lanes of the substrate was drained, the lanes were rinsed with 1 mL of PC buffer per lane, and the liquid in the lanes was drained after rinsing;
   3) 396 µL of PC buffer and 4 µL of a 100 mM NHS-PEG₄-N₃ solution were mixed. The mixture was vortexed for mixing, and then centrifuged for 2 s;
   4) The solution prepared in the step 3) was immediately introduced into lanes of the substrate with 25 µL per lane. The substrate was incubated for 1 h at room temperature;
   5) After the reaction, the substrate was washed with washing solution 1, RI02 and ultrapure water, each 1 mL, on the fluid instrument;
   6) The liquid in the lanes of the substrate was drained on the fluid instrument. 497.25 µL of formamide was added into a 1.5-mL centrifuge tube before 1.75 µL of DIPEA and 1 µL of acetic anhydride were sequentially added. The mixture was vortexed for mixing, centrifuged for 2 s, and immediately introduced into the lanes of the substrate with 25 µL per lane. The substrate was incubated for 15 min at room temperature. After the reaction, the substrate was washed with washing solution 1, RI02 and ultrapure water, each 1 mL, on the fluid instrument.
6. Probe immobilization:
   1) On the fluid instrument, liquid in the lanes of the substrate was drained, the lanes were rinsed with 1 mL of immobilization solution per lane, and the liquid in the lanes was drained after rinsing;
   2) 360 µL of the immobilization solution and 40 µL of a 100 µM A30 solution were mixed. The mixture was vortexed for mixing, centrifuged for 2 s, and immediately introduced into the lanes of the substrate with 25 µL per lane. The substrate was incubated for 16 h at 37 °C;
   3) After the reaction, when the substrate returned to room temperature, the substrate was washed with washing solution 1 and RI02, each 1 mL, on a fluid instrument.

The functional group-modified substrate can be soaked in RI02 solution for storage at 4 °C for 1 year.

### Example 3

The specific procedures for the coating epoxy-modified substrate with PamamDendrimer (polyamidoamine dendrimer) and modifying the PamamDendrimer-coated substrate with functional groups are as follows:
1. The 5% PamamDendrimer solution was stored at room temperature for 15 min. 380 µL of SC buffer solution was added into 200 µL of the PamamDendrimer solution. The mixture was well mixed by vortex, and centrifuged for 2 s.
2. The solution prepared in step 1 was introduced into lanes of an epoxy-modified substrate with 25 µL of solution into each lane. The lane ports were sealed (to prevent the solution from volatilizing). The substrate was incubated at 37 °C for 16 h.
3. After the reaction, when the substrate returned to room temperature, the substrate was washed with washing solution 1 and RI02, each 1 mL, on a fluid instrument. The parameters of the fluid instrument were: reagent amount = 1000, speed = 10, circle = 1, delay = 0, number = 1.
4. The PamamDendrimer-coated substrate was soaked in RI02 solution for storage at 4 °C for 1 week.
5. The PamamDendrimer-coated substrate was modified with functional groups, which refer to groups that can participate in subsequent reactions, and may be -N₃, -DBCO and the like. In this example, the functional group was -N₃. The specific procedures of the functional group modification are as follows:
   1) An NHS-PEG₄-N₃ solution with a final concentration of 100 mM was prepared with the solvent being anhydrous DMSO (anhydrous dimethyl sulfoxide);
   2) A solution containing NHS-PEG₄-N₃ was introduced into the lanes of the PLO-coated substrate. On the fluid instrument, liquid in the lanes of the substrate was drained, the lanes were rinsed with 1 mL of PC buffer per lane, and the liquid in the lanes was drained after rinsing;
   3) 396 µL of PC buffer and 4 µL of a 100 mM NHS-PEG₄-N₃ solution were mixed. The mixture was vortexed for mixing, and then centrifuged for 2 s;
   4) The solution prepared in the step 3) was immediately introduced into lanes of the substrate with 25 µL per lane. The substrate was incubated for 1 h at room temperature;
   5) After the reaction, the substrate was washed with washing solution 1, RI02 and ultrapure water, each 1 mL, on the fluid instrument.
6. Probe immobilization:
   1) On the fluid instrument, liquid in the lanes of the substrate was drained, the lanes were rinsed with 1 mL of immobilization solution per lane, and the liquid in the lanes was drained after rinsing;
   2) 360 µL of the immobilization solution and 40 µL of a 100 µM A30 solution were mixed. The mixture was vortexed for mixing, centrifuged for 2 s, and immediately introduced into the lanes of the substrate with 25 µL per lane. The substrate was incubated for 16 h at 37 °C;
   3) After the reaction, when the substrate returned to room temperature, the substrate was washed with washing solution 1 and RI02, each 1 mL, on a fluid instrument;
   4) The liquid in the lanes of the substrate was drained on the fluid instrument. 497.25 µL of formamide was added into a 1.5-mL centrifuge tube before 1.75 µL of DIPEA and 1 µL of acetic anhydride were sequentially added. The mixture was vortexed for mixing, centrifuged for 2 s, and immediately introduced into the lanes of the substrate with 25 µL per lane. The substrate was incubated for 15 min at room temperature. After the reaction, the substrate was washed with washing solution 1, RI02 and ultrapure water, each 1 mL, on the fluid instrument.

The functional group-modified substrate can be soaked in RI02 solution for storage at 4 °C for 1 year.

### Example 4

The specific procedures for coating the epoxy-modified substrate with PEI (polyethyleneimine) and modifying the PEI-coated substrate with functional groups are as follows:
1. The 1% PEI solution was stored at room temperature for 15 min. 380 µL of SC buffer solution was added into 200 µL of the PEI solution. The mixture was well mixed by vortex, and centrifuged for 2 s.
2. The solution prepared in step 1 was introduced into lanes of an epoxy-modified substrate with 25 µL of solution into each lane. The lane ports were sealed (to prevent the solution from volatilizing). The substrate was incubated at 37 °C for 16 h.
3. After the reaction, when the substrate returned to room temperature, the substrate was washed with washing solution 1 and RI02, each 1 mL, on a fluid instrument. The parameters of the fluid instrument were: reagent amount = 1000, speed = 10, circle = 1, delay = 0, number = 1.
4. The PEI-coated substrate was soaked in RI02 solution for storage at 4 °C for 1 week.
5. The PEI-coated substrate was modified with functional groups, which refer to groups that can participate in subsequent reactions, and may be -N₃, -DBCO and the like. In this example, the functional group was -N₃. The specific procedures of the functional group modification are as follows:
   1) An NHS-PEG₄-N₃ solution with a final concentration of 100 mM was prepared with the solvent being anhydrous DMSO (anhydrous dimethyl sulfoxide);
   2) A solution containing NHS-PEG₄-N₃ was introduced into the lanes of the PEI-coated substrate. On the fluid instrument, liquid in the lanes of the substrate was drained, the lanes were rinsed with 1 mL of PC buffer per lane, and the liquid in the lanes was drained after rinsing;
   3) 396 µL of PC buffer and 4 µL of a 100 mM NHS-PEG₄-N₃ solution were mixed. The mixture was vortexed for mixing, and then centrifuged for 2 s;
   4) The solution prepared in the step 3) was immediately introduced into lanes of the substrate with 25 µL per lane. The substrate was incubated for 1 h at room temperature;
   5) After the reaction, the substrate was washed with washing solution 1, RI02 and ultrapure water, each 1 mL, on the fluid instrument.
   6) The liquid in the lanes of the substrate was drained on the fluid instrument. 497.25 µL of formamide was added into a 1.5-mL centrifuge tube before 1.75 µL of DIPEA and 1 µL of acetic anhydride were sequentially added. The mixture was vortexed for mixing, centrifuged for 2 s, and immediately introduced into the lanes of the substrate with 25 µL per lane. The substrate was incubated for 15 min at room temperature. After the reaction, the substrate was washed with washing solution 1, RI02 and ultrapure water, each 1 mL, on the fluid instrument.
6. Probe immobilization:
   1) On the fluid instrument, liquid in the lanes of the substrate was drained, the lanes were rinsed with 1 mL of immobilization solution per lane, and the liquid in the lanes was drained after rinsing;
   2) 360 µL of the immobilization solution and 40 µL of a 100 µM A30 solution were mixed. The mixture was vortexed for mixing, centrifuged for 2 s, and immediately introduced into the lanes of the substrate with 25 µL per lane. The substrate was incubated for 16 h at 37 °C;
   3) After the reaction, when the substrate returned to room temperature, the substrate was washed with washing solution 1 and RI02, each 1 mL, on a fluid instrument.

The functional group-modified substrate can be soaked in RI02 solution for storage at 4 °C for 1 year.

### Example 5

The specific procedures for the coating epoxy-modified substrate with chitosan and modifying the chitosan-coated substrate with functional groups are as follows:
1. The 1% chitosan solution was stored at room temperature for 15 min. 380 µL of SC buffer solution was added into 20 µL of the chitosan solution. The mixture was well mixed by vortex, and centrifuged for 2 s.
2. The solution prepared in step 1 was introduced into lanes of an epoxy-modified substrate with 25 µL of solution into each lane. The lane ports were sealed (to prevent the solution from volatilizing). The substrate was incubated at 37 °C for 16 h.
3. After the reaction, when the substrate returned to room temperature, the substrate was washed with washing solution 1 and RI02, each 1 mL, on a fluid instrument. The parameters of the fluid instrument were: reagent amount = 1000, speed = 10, circle = 1, delay = 0, number = 1.
4. The chitosan-coated substrate was soaked in RI02 solution for storage at 4 °C for 1 week.
5. The chitosan-coated substrate was modified with functional groups, which refer to groups that can participate in subsequent reactions, and may be -N₃, -DBCO and the like. In this example, the functional group was -N₃. The specific procedures of the functional group modification are as follows:
   1) An NHS-PEG₄-N₃ solution with a final concentration of 100 mM was prepared with the solvent being anhydrous DMSO (anhydrous dimethyl sulfoxide);
   2) A solution containing NHS-PEG₄-N₃ was introduced into the lanes of the PLO-coated substrate. On the fluid instrument, liquid in the lanes of the flowcell was drained, the lanes were rinsed with 1 mL of PC buffer per lane, and the liquid in the lanes was drained after rinsing;
   3) 396 µL of PC buffer and 4 µL of a 100 mM NHS-PEG₄-N₃ solution were mixed. The mixture was vortexed for mixing, and then centrifuged for 2 s;
   4) The solution prepared in the step 3) was immediately introduced into lanes of the flowcell with 25 µL per lane. The substrate was incubated for 1 h at room temperature;
   5) After the reaction, the substrate was washed with washing solution 1, RI02 and ultrapure water, each 1 mL, on the fluid instrument.
   6) The liquid in the lanes of the substrate was drained on the fluid instrument. 497.25 µL of formamide was added into a 1.5-mL centrifuge tube before 1.75 µL of DIPEA and 1 µL of acetic anhydride were sequentially added. The mixture was vortexed for mixing, centrifuged for 2 s, and immediately introduced into the lanes of the substrate with 25 µL per lane. The substrate was incubated for 15 min at room temperature. After the reaction, the substrate was washed with washing solution 1, RI02 and ultrapure water, each 1 mL, on the fluid instrument.
6. Probe immobilization:
   1) On the fluid instrument, liquid in the lanes of the substrate was drained, the lanes were rinsed with 1 mL of immobilization solution per lane, and the liquid in the lanes was drained after rinsing;
   2) 360 µL of the immobilization solution and 40 µL of a 100 µM A30 solution were mixed. The mixture was vortexed for mixing, centrifuged for 2 s, and immediately introduced into the lanes of the substrate with 25 µL per lane. The substrate was incubated for 16 h at 37 °C;
   3) After the reaction, when the substrate returned to room temperature, the substrate was washed with washing solution 1 and RI02, each 1 mL, on a fluid instrument.

The functional group-modified substrate can be soaked in RI02 solution for storage at 4 °C for 1 year.

### Example 6

In this example, the chips prepared in Examples 1 to 5 were subjected to a probe density detection. The specific detection procedures are as follows:
1. T20-CY3 hybridization: liquid in the lanes of the chips prepared in examples 1 to 5 was drained on the fluid instrument. 20 µL of hybridization solution A (3× SSC (pH 7.2 to 7.4) containing 3 µM T20-CY3) was introduced into each lane, and the chips were incubated at 55 °C for 15 min for hybridization. After cooling to room temperature, the chips were washed with 1 mL of RI02 on the fluid instrument. The Cy3 in the T20-CY3 was located at the 5' end of the T20 sequence.

Detection: Images were taken on a fluorescence detection system using a 20-fold objective lens, with a wavelength of 532 nm, a laser power of 35 mW, an exposure time of 60 ms, and were analyzed using ImageJ.

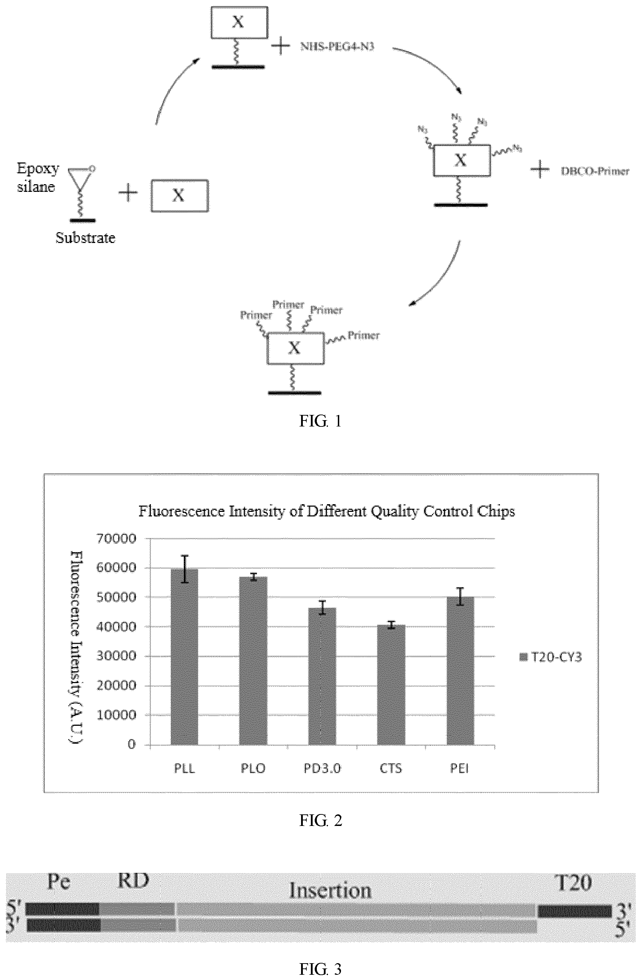

The probe density detection results of the chips prepared in Examples 1 to 5 were shown in FIG 2, where the ordinate represents the fluorescence intensity (A.U.) in unit dot/FOV, i.e., the mean fluorescence intensity of 1 observation region of 110 × 110 µm, and PLL, PLO, PD3.0, CTS and PEI on the abscissa respectively correspond to the chips modified with polymers PLL, PLO, PD3.0 (polyamidoamine dendrimer), CTS (chitosan) and PEI.

### Example 7

Library hybridization and detection were performed using the chips prepared in Examples 1 to 5. The specific procedures are as follows:
1. DNA hybridization library preparation

DNA library: DNA library of fragments with length of 150 to 300 bp and known sequences at both ends, the molecular structure of the library is shown in FIG 3; Insertion: an inserted fragment derived from phi-X174 standard strain; T20, Pe and RD are respectively sequences set forth in SEQ ID Nos. 2 to 4.

The DNA library was mixed with 52 µL of deionized water before 18 µL of freshly prepared 0.2 M NaOH solution was added. After well mixing, the mixture was let stand for denaturation at room temperature for 8 min, and then the reaction was stopped by adding 20 µL of 400 mM Tris-HCl buffer, pH 8.0 to obtain 100 µL of 100 pM denatured DNA library. 2. Hybridization of denatured DNA library with chip probes

The denatured DNA library was diluted to 5 pM using a hybridization solution containing 3× SSC (20× SSC buffer diluted with RNase-free water), pH 7.3. The diluted DNA library was introduced into lanes of the chip and the chip was incubated for hybridization at 42 °C for 30 min. 160 to 260 µL of washing reagent (5× SSC, 0.05% Tween 20, pH 7.0) was introduced at a rate of 250 µL/min. The hybridization reaction was thus completed.

### 3. Initial extension of the template

1) 160 to 260 µL of extension buffer reagent (20 mM tris(hydroxymethyl)aminomethane (Tris), 10 mM ammonium sulfate, 2 mM magnesium sulfate, 1.5 M betaine, 1.3% dimethyl sulfoxide, 0.45 M N-methyl formamide, 1.5 M carboxamide, and 0.1% TritonX-100, pH 9.0) was introduced into the lanes of the chip at a rate of 500 µL/min;
2) 160 to 260 µL of extension reagent (extension buffer reagent, 3 µg/mL Bst DNA polymerase, 200 µM dNTPs) was introduced into the lanes of the chip at a rate of 500 µL/min, and the chip was incubated at 50 to 60 °C for 5 min to complete initial extension of the template.

### 4. DNA cluster generation

The amplification was carried out using the template walking technique disclosed in the article Isothermal amplification method for next-generation sequencing (Zhaochun Ma, et al, et.al, PNAS August 27, 2013 110(35):14320-14323, https://doi.org/10.1073/pnas.1311334110).

### 5. DNA cluster detection

1) The thermal cycle temperature was set to 50 to 60 °C;
2) 160 to 260 µL of denaturing reagent formamide was introduced into lanes of the chip at a rate of 500 µL/min. The chip was incubated for 5 min to break the DNA double-helix structure;
3) 160 to 260 µL of quality control reagent (0.5 µM RD-Cy3, 3× SSC) was introduced into lanes of the chip at a rate of 500 µL/min, where Cy3 in RD-Cy3 was located at the 5' end of RD sequence;
4) The thermal cycle temperature was set to 25 °C and the chip was incubated for 15 to 30 min for reaction;
5) 160 to 260 µL of washing reagent was introduced into lanes of the chip at a rate of 500 µL/min;
6) Images were taken on a fluorescence detection system using a 20-fold objective lens, with a wavelength of 532 nm, a laser power of 300 mW, and an exposure time of 20 ms.

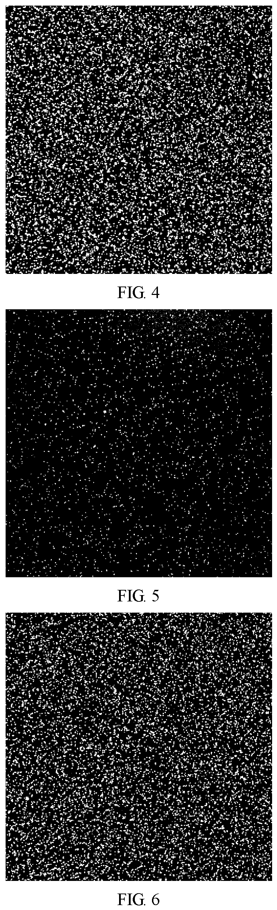

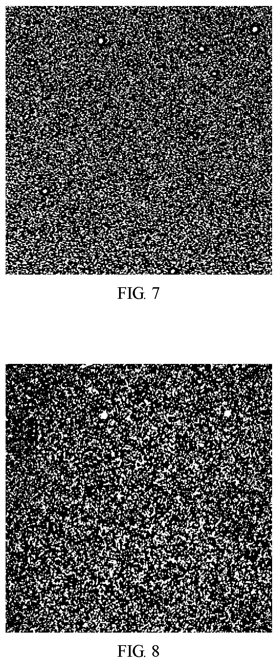

The results of DNA cluster detection are shown in FIGs. 3 to 7 and Table 1, and the results of DNA cluster detection corresponding to chips prepared in Examples 1 to 5 are shown in FIGs. 4 to 8, respectively.

**Table 1.**

| Chip | Density (cluster/µm²) | Density (cluster/mm²) | Average luminance per cluster (cts) |
|---|---|---|---|
| Chip prepared in Example 1 | 0.07187 | 71870 | 1351 |
| Chip prepared in Example 2 | 0.01711 | 17110 | 743 |
| Chip prepared in Example 3 | 0.077666 | 77666 | 882 |
| Chip prepared in Example 4 | 0.143442 | 143442 | 3857 |
| Chip prepared in Example 5 | 0.106125329 | 106125 | 4336 |

As can be seen from the detection results, the chips prepared in Examples 1 to 5 were successfully used for hybridization of the library and DNA cluster generation.

Technical features in the above examples may be combined in any combinations. In order to make the description brief, all possible combinations of various technical features in the above examples are not described; however, it should be considered as being within the scope of this specification as long as there is no contradiction in the combinations of the technical features.

The above examples only illustrate one or more embodiments of the present disclosure for the purpose of specific and detailed description, but should not be construed as limiting the scope of the present disclosure. Therefore, the protection scope of the present disclosure should be determined with reference to the appended claims.

## Claims

1. An amino-modified chip, comprising:
a substrate modified with an epoxy group;
a polymer attached to the substrate via the epoxy group, wherein at least one structural unit of the polymer comprises an amino group, and the amino group is a primary amino group and/or a secondary amino group; and
a probe attached to the polymer, wherein the probe is attached to the polymer via a linker compound, and the linker compound has a molecular structure comprising a first linker compound and a second linker compound; the first linker compound is bonded to the probe, and the second linker compound is attached to the polymer through the amino group,
wherein the second linker compound is selected from at least one of an -NHS group, an epoxy group and an isocyanate group, wherein the second linker compound is made of NHS-PEGₙ-DBCO or NHS-PEGₙ-N₃, wherein n is 3 to 2000, and group -NHS in NHS-PEGₙ-DBCO or NHS-PEGₙ-N₃ is attached to the amino group via reaction.

2. The chip according to claim 1, wherein in the polymer, each of the structural unit comprises an average of 0.05 to 10 identical or different amino groups, wherein optionally
in the polymer, each of the structural unit comprises an average of 1 to 5 identical or different amino groups.

3. The chip according to any of claims 1 or 2 , wherein the polymer has a degree of polymerization in a range of 10 to 5000, wherein optionally
in the polymer the structural unit comprises at least one of

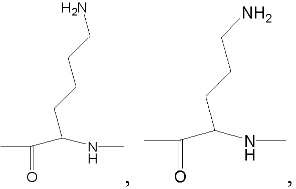

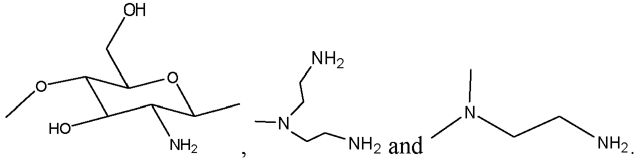

4. The chip according to any of claims 1-3, wherein the polymer is selected from at least one of a polylysine, a polyornithine, a chitosan, a polyamidoamine dendrimer, and polyethyleneimine.

5. The chip according to claims 1-3, wherein the probe is modified with group -DBCO or group -N₃; the bonding is a covalent bonding between group -DBCO and group -N₃.

6. A method for preparing a chip, comprising:
(1) acquiring a substrate modified with an epoxy group;
(2) attaching a polymer to the substrate via reaction, wherein at least one structural unit of the polymer comprises an amino group, and the amino group is a primary amino group or a secondary amino group; and
(3) attaching a probe to the polymer, wherein the probe in step (3) is attached to the polymer via a linker compound, and the linker compound has a molecular structure comprising a first linker compound and a second linker compound; the first linker compound is bonded to the probe, and the second linker compound is attached to the polymer through the amino group, wherein the second linker compound is selected from at least one of an -NHS group, an epoxy group and an isocyanate group, the linker compound is made of NHS-PEGₙ-DBCO or NHS-PEGₙ-N₃, wherein n is 3 to 2000, and group -NHS in NHS-PEGₙ-DBCO or NHS-PEGₙ-N₃ is attached to the amino group via reaction.

7. The method according to claim 6, wherein I) in the polymer, each of the structural unit comprises an average of 0.05 to 10 identical or different amino groups, wherein optionally in the polymer, each of the structural unit comprises an average of 1 to 5 identical or different amino groups and/or
II) the polymer has a degree of polymerization in a range of 10 to 5000

8. The method according to claim 7, wherein in the polymer the structural unit comprises at least one of 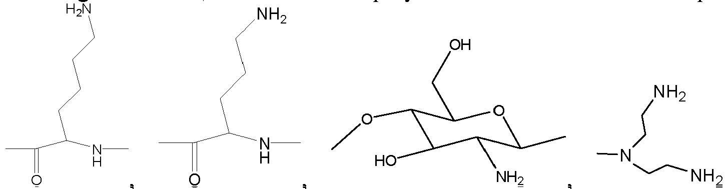 and 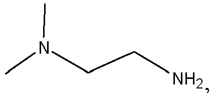 and/or
III) the polymer is selected from at least one of polylysine, polyornithine, chitosan, a polyamidoamine dendrimer and polyethyleneimine.

9. The method according to claim 8, wherein IV) in step (2), the attaching via reaction is conducted by contacting the polymer with the substrate in an alkaline solution at pH 8.5 to 10; and/or
V) in step (2), the attaching via reaction is conducted at a reaction temperature of 37 to 55 °C for 3 to 24 h and/or
VI) the method further comprises the step (3): attaching a probe to the polymer.

10. The method according to claim 9, wherein the probe is modified with group -DBCO or group -N₃; the bonding is a covalent bonding between group -DBCO and group -N₃.

11. The method according to any of claims 6-10, wherein the attaching via reaction is conducted by contacting the linker compound with the polymer in an alkaline solution at pH 7 to 9.

12. The method according to claim 10, wherein the attaching via reaction is conducted at room temperature for 30 to 90 min.

13. The method according to any of claims 6-11, wherein the covalent bonding is conducted by contacting the probe with the linker compound in an alkaline solution at pH 7 to 8, wherein optionally the alkaline solution for covalent bonding comprises a surfactant selected from at least one of tetradecyltrimethylammonium bromide, cetyltrimethylammonium bromide and dodecyltrimethylammonium bromide.

14. Use of the chip according to any of claims 1-5 or a chip prepared by the method according to any of claims 6-13 in preparation or analysis of a biomolecule.

## Patentansprüche

1. Aminomodifizierter Chip, umfassend:
ein Substrat, das mit einer Epoxidgruppe modifiziert ist;
ein Polymer, das über die Epoxidgruppe an das Substrat gebunden ist, wobei mindestens eine Struktureinheit des Polymers eine Aminogruppe umfasst, und die Aminogruppe eine primäre Aminogruppe und/oder eine sekundäre Aminogruppe ist; und
eine Sonde, die an das Polymer gebunden ist, wobei die Sonde über eine Linkerverbindung an das Polymer gebunden ist, und die Linkerverbindung eine Molekularstruktur aufweist, die eine erste Linkerverbindung und eine zweite Linkerverbindung umfasst; die erste Linkerverbindung ist an die Sonde gebunden, und die zweite Linkerverbindung ist über die Aminogruppe an das Polymer gebunden,
wobei die zweite Linkerverbindung aus mindestens einer -NHS-Gruppe, einer Epoxidgruppe und einer Isocyanatgruppe ausgewählt ist, wobei die zweite Linkerverbindung aus NHS-PEGₙ--DBCO oder NHS-PEGₙ-N₃ hergestellt ist, wobei n 3 bis 2000 ist, und die Gruppe -NHS in NHS-PEGₙ-DBCO oder NHS-PEGₙ-N₃ durch Reaktion an die Aminogruppe gebunden ist.

2. Chip nach Anspruch 1, wobei in dem Polymer jede der Struktureinheiten durchschnittlich 0,05 bis 10 identische oder unterschiedliche Aminogruppen umfasst, wobei optional
in dem Polymer jede der Struktureinheiten aus durchschnittlich 1 bis 5 identischen oder unterschiedlichen Aminogruppen besteht.

3. Chip nach einem der Ansprüche 1 oder 2, wobei das Polymer einen Polymerisationsgrad in einem Bereich von 10 bis 5000 aufweist, wobei optional
im Polymer umfasst die Struktureinheit mindestens eine von

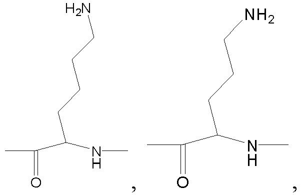

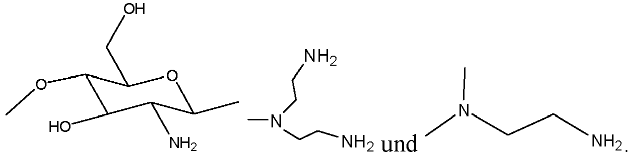

4. Chip nach einem der Ansprüche 1 bis 3, wobei das Polymer ausgewählt ist aus mindestens einem Polylysin, einem Polyornithin, einem Chitosan, einem Polyamidoamin-Dendrimer und Polyethylenimin.

5. Chip nach Anspruch 1 bis 3, wobei die Sonde mit der Gruppe -DBCO oder der Gruppe -N₃ modifiziert ist; die Bindung ist eine kovalente Bindung zwischen der Gruppe -DBCO und der Gruppe -N₃.

6. Verfahren zum Herstellen eines Chips, umfassend:
(1) Erwerb eines mit einer Epoxidgruppe modifizierten Substrats;
(2) Anhängen eines Polymers an das Substrat durch Reaktion, wobei mindestens eine Struktureinheit des Polymers eine Aminogruppe umfasst und die Aminogruppe eine primäre Aminogruppe oder eine sekundäre Aminogruppe ist; und
(3) Anhängen einer Sonde an das Polymer, wobei die Sonde in Schritt (3) über eine Linkerverbindung an das Polymer gebunden ist, und die Linkerverbindung eine Molekularstruktur aufweist, die eine erste Linkerverbindung und eine zweite Linkerverbindung umfasst; die erste Linkerverbindung ist an die Sonde gebunden, und die zweite Linkerverbindung ist durch die Aminogruppe an das Polymer gebunden,
wobei die zweite Linkerverbindung aus mindestens einer -NHS-Gruppe, einer Epoxidgruppe und einer Isocyanatgruppe ausgewählt ist, wobei die Linkerverbindung aus NHS-PEGₙ-DBCO oder NHS-PEGₙ-N₃ hergestellt ist, wobei n 3 bis 2000 ist, und die Gruppe -NHS in NHS-PEGₙ-DBCO oder NHS-PEGₙ-N₃ durch Reaktion an die Aminogruppe gebunden ist.

7. Verfahren nach Anspruch 6, wobei i) in dem Polymer jede der Struktureinheiten durchschnittlich 0,05 bis 10 identische oder unterschiedliche Aminogruppen umfasst, wobei optional in dem Polymer jede der Struktureinheiten durchschnittlich 1 bis 5 identische oder unterschiedliche Aminogruppen umfasst und/oder
II) das Polymer hat einen Polymerisationsgrad in einem Bereich von 10 bis 5000.

8. Verfahren nach Anspruch 7, wobei in dem Polymer die Struktureinheit mindestens eine von 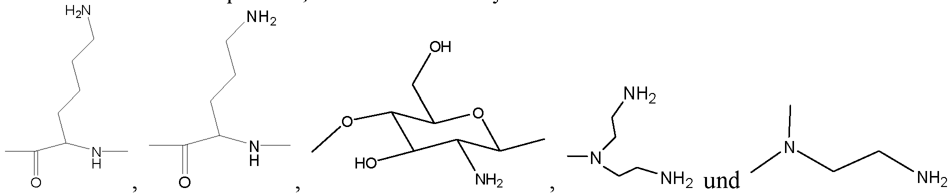 umfasst, und/oder
III) das Polymer ist ausgewählt aus mindestens einem Polylysin, Polyornithin, Chitosan, einem Polyamidoamin-Dendrimer und Polyethylenimin.

9. Verfahren nach Anspruch 8, wobei IV) in Schritt (2) die Bindung über die Reaktion durch Kontaktierung des Polymers mit dem Substrat in einer alkalischen Lösung bei pH 8,5 bis 10 durchgeführt wird; und/oder
V) in Schritt (2) wird das Anhängen über die Reaktion bei einer Reaktionstemperatur von 37 bis 55 ° C für 3 bis 24 h durchgeführt und/oder
VI) Das Verfahren umfasst weiterhin den Schritt (3): das Anbringen einer Sonde an dem Polymer.

10. Verfahren nach Anspruch 9, wobei die Sonde mit der Gruppe -DBCO oder der Gruppe -N₃ modifiziert wird; die Bindung ist eine kovalente Bindung zwischen der Gruppe -DBCO und der Gruppe -N₃.

11. Verfahren nach einem der Ansprüche 6 bis 10, wobei die Bindung über die Reaktion durch Kontaktierung der Linkerverbindung mit dem Polymer in einer alkalischen Lösung bei pH 7 bis 9 durchgeführt wird.

12. Verfahren nach Anspruch 10, wobei die Attach-via-Reaktion bei Raumtemperatur für 30 bis 90 min durchgeführt wird.

13. Verfahren nach einem der Ansprüche 6 bis 11, wobei die kovalente Bindung durch Kontaktierung der Sonde mit der Linkerverbindung in einer alkalischen Lösung bei pH 7 bis 8 durchgeführt wird,
wobei optional die alkalische Lösung für die kovalente Bindung ein Tensid umfasst, das ausgewählt ist aus mindestens einem von Tetradecyltrimethylammoniumbromid, Cetyltrimethylammoniumbromid und Dodecyltrimethylammoniumbromid.

14. Verwendung des Chips nach einem der Ansprüche 1 bis 5 oder eines Chips, der nach dem Verfahren nach einem der Ansprüche 7 bis 13 hergestellt wurde, zur Vorbereitung oder Analyse eines Biomoleküls.

## Revendications

1. Une puce amino-modifiée, comprenant :
un substrat modifié avec un groupe époxy ;
un polymère fixé au substrat par l'intermédiaire du groupe époxy, dans lequel au moins une unité structurelle du polymère comprend un groupe aminé, et le groupe amino est un groupe aminé primaire et/ou un groupe aminé secondaire ; et
une sonde fixée au polymère, dans laquelle la sonde est fixée au polymère par l'intermédiaire d'un composé de liaison, et le composé de liaison a une structure moléculaire comprenant un premier composé de liaison et un second composé de liaison ; le premier composé de liaison est lié à la sonde, et le second composé de liaison est attaché au polymère par l'intermédiaire du groupe amino,
dans lequel le deuxième composé de liaison est choisi parmi au moins un d'un groupe -NHS, d'un groupe époxy et d'un groupe isocyanate, dans lequel le deuxième composé de liaison est constitué de NHS-PEGₙ-DBCO ou NHS-PEGₙ-N₃, dans lequel n est de 3 à 2000, et le groupe - NHS dans NHS-PEGₙ-DBCO ou NHS-PEGₙ-N₃ est attaché au groupe amino par réaction.

2. Puce selon la revendication 1, dans laquelle dans le polymère, chacune des unités structurelles comprend une moyenne de 0,05 à 10 groupes aminés identiques ou différents, dans laquelle éventuellement
Dans le polymère, chacune des unités structurelles comprend en moyenne 1 à 5 groupes aminés identiques ou différents.

3. Puce selon l'une quelconque des revendications 1 ou 2, dans laquelle le polymère a un degré de polymérisation dans une plage de 10 à 5000, dans laquelle éventuellement
Dans le polymère, l'unité structurelle comprend au moins l'un des éléments suivants

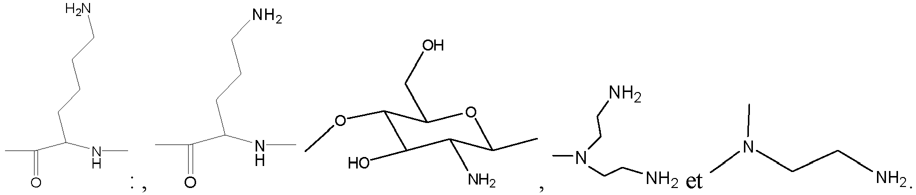

4. Puce selon l'une quelconque des revendications 1 à 3, dans laquelle le polymère est choisi parmi au moins l'un des suivants : polylysine, polyornithine, chitosane, dendrimère de polyamidoamine et polyéthylèneimine.

5. Planche à billets Puce selon les revendications 1 à 3, dans laquelle la sonde est modifiée avec le groupe -DBCO ou le groupe -N₃ ; la liaison est une liaison covalente entre le groupe -DBCO et le groupe -N₃.

6. Planche à voile Procédé de préparation d'une puce, comprenant :
(1) l'acquisition d'un substrat modifié avec un groupe époxy ;
(2) fixation d'un polymère au substrat par réaction, dans laquelle au moins une unité structurelle du polymère comprend un groupe aminé, et le groupe amino est un groupe amino primaire ou un groupe aminé secondaire ; et
(3) fixation d'une sonde au polymère, dans laquelle la sonde de l'étape (3) est fixée au polymè re par l'intermédiaire d'un composé de liaison, et le composé de liaison a une structure moléculaire comprenant un premier composé de liaison et un second composé de liaison ; le premier composé de liaison est lié à la sonde, et le deuxième composé de liaison est attaché au polymère par le biais du groupe amino,
dans lequel le deuxième composé de liaison est choisi parmi au moins un d'un groupe -NHS, d'un groupe époxy et d'un groupe isocyanate, le composé de liaison est constitué de NHS-PEGₙ-DBCO ou NHS-PEGₙ-N₃, dans lequel n est de 3 à 2000, et le groupe -NHS de NHS-PEGₙ-DBCO ou NHS-PEGₙ-N₃ est attaché au groupe amino par réaction.

7. Aéroport Procédé selon la revendication 6, dans lequel I) dans le polymère, chacune des unités structurelles comprend une moyenne de 0,05 à 10 groupes aminés identiques ou différents, dans lequel, facultativement, dans le polymère, chacune des unités structurelles comprend une moyenne de 1 à 5 groupes aminés identiques ou différents et/ou II) le polymère a un degré de polymérisation compris entre 10 et 5000.

8. Épisode 8 Procédé selon la revendication 7, dans lequel dans le polymère l'unité structurelle comprend au moins l'un des éléments suivants : 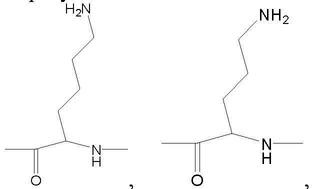 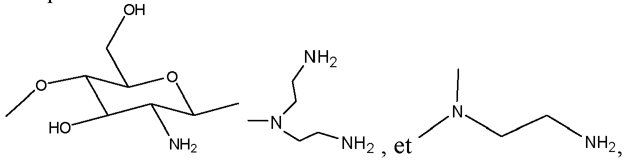 et/ou
III) le polymère est choisi parmi au moins l'un des suivants : polylysine, polyornithine, chitosane, un dendrimère de polyamidoamine et polyéthylèneimine.

9. Planche à billets Procédé selon la revendication 8, dans lequel IV) à l'étape (2), la fixation par réaction est effectuée en mettant en contact le polymère avec le substrat dans une solution alcaline à un pH de 8,5 à 10 ; et/ou
V) à l'étape (2), la fixation par réaction est effectuée à une température de réaction de 37 à 55 ° C pendant 3 à 24 h et/ou
VI) le procédé comprend en outre l'étape (3) : la fixation d'une sonde sur le polymère.

10. Aéroport international Procédé selon la revendication 9, dans lequel la sonde est modifiée avec le groupe -DBCO ou le groupe -N₃ ; la liaison est une liaison covalente entre le groupe - DBCO et le groupe -N₃.

11. Aéroport international Procédé selon l'une quelconque des revendications 6 à 10, dans lequel la fixation par réaction est effectuée par contact avec le composé de liaison avec le polymère dans une solution alcaline à pH 7 à 9.

12. Épisode 12 Procédé selon la revendication 10, dans lequel la fixation par réaction est effectuée à température ambiante pendant 30 à 90 min.

13. Planche à billets Procédé selon l'une quelconque des revendications 6 à 11, dans lequel la liaison covalente est réalisée par contact avec la sonde avec le composé de liaison dans une solution alcaline à pH 7 à 8,
dans lequel, de manière facultative, la solution alcaline pour liaison covalente comprend un tensioactif choisi parmi au moins un bromure de tétradécyltriméthylammonium, le bromure de cétyltriméthylammonium et le bromure de dodécyltriméthylammonium.

14. Planche à billets Utilisation de la puce selon l'une quelconque des revendications 1 à 5 ou d'une puce préparée par le procédé selon l'une quelconque des revendications 7 à 13 dans la préparation ou l'analyse d'une biomolécule.
